# EUROPEAN PATENT SPECIFICATION

(11) **EP 3 458 854 B1**
(45) Date of publication and mention of the grant of the patent: **22.11.2023**
(21) Application number: 17723135.4
(22) Date of filing: 17.05.2017
(51) Int. Cl.: G01N 33/50, G01N 33/543

(54) **METHOD AND KIT FOR CAPTURING EXTRACELLULAR VESICLES (EVS) ON A SOLID SURFACE**
VERFAHREN UND KIT ZUR ERFASSUNG EXTRAZELLULÄRER VESIKEL (EVS) AUF EINER FESTEN OBERFLÄCHE
PROCÉDÉ ET KIT PERMETTANT DE CAPTURER DES VÉSICULES EXTRACELLULAIRES SUR UNE SURFACE SOLIDE

(30) Priority: 20.05.2016 EP 16170645
(43) Date of publication of application: 27.03.2019
(73) Proprietor: Unicyte EV AG, 6370 Oberdorf (CH)
(72) Inventor: DEREGIBUS, Maria Chiara, 10132 Torino (IT); BUSSOLATI, Benedetta, 10126 Torino (IT); CAMUSSI, Giovanni, 10132 Torino (IT); DIMUCCIO, Veronica, 10128 Torino (IT)
(74) Representative: Comoglio, Elena
(86) International application number: PCT/EP2017/061811
(87) International publication number: WO 2017/198695

(56) References cited:
- US-A1- 2013 273 544
- US-A1- 2013 337 440
- JOEL Z. NORDIN ET AL: "Ultrafiltration with size-exclusion liquid chromatography for high yield isolation of extracellular vesicles preserving intact biophysical and functional properties", NANOMEDICINE: NANOTECHNOLOGY, BIOLOGY AND MEDICINE, vol. 11, no. 4, 1 May 2015 (2015-05-01), pages 879-883, XP055286861, NL ISSN: 1549-9634, DOI: 10.1016/j.nano.2015.01.003
- LEONORA BALAJ ET AL: "Heparin affinity purification of extracellular vesicles", SCIENTIFIC REPORTS, vol. 5, 19 May 2015 (2015-05-19), page 10266, XP055286185, DOI: 10.1038/srep10266
- MALENE MØLLER JØRGENSEN ET AL: "Potentials and capabilities of the Extracellular Vesicle (EV) Array", JOURNAL OF EXTRACELLULAR VESICLES, vol. 4, no. 0, 8 April 2015 (2015-04-08), page 26048, XP055286209, DOI: 10.1681/ASN.2009121238
- VERONICA DIMUCCIO ET AL: "Urinary CD133+ Extracellular Vesicles Are Decreased in Kidney Transplanted Patients with Slow Graft Function and Vascular Damage", PLOS ONE, vol. 9, no. 8, 6 August 2014 (2014-08-06), page e104490, XP055286225, DOI: 10.1371/journal.pone.0104490
- SURI C ET AL: "Determination of immunoglobulin M concentration by piezoelectric crystal immunobiosensor coated with protamine", BIOSENSORS AND BIOELECTRONICS, ELSEVIER BV, NL, vol. 9, no. 7, 1 January 1994 (1994-01-01) , pages 535-542, XP026597701, ISSN: 0956-5663, DOI: 10.1016/0956-5663(94)90016-7 [retrieved on 1994-01-01]
- Kosanovic Maja ET AL: "Isolation of urinary extracellular vesicles from Tamm- Horsfall protein-depleted urine and their application in the development of a lectin-exosome-binding assay", Biotechniques, vol. 57, no. 3, 1 September 2014 (2014-09-01), pages 143-149, XP055909745, US ISSN: 0736-6205, DOI: 10.2144/000114208

## Description

The present invention relates to methods and a kit for capturing extracellular vesicles (EVs) from a biological fluid sample and binding them to a solid substrate. The methods and kit of the invention are useful for the detection and quantification of EVs in biological fluid samples, particularly within the context of diagnostic applications.

Vesicular-mediated communication between cells appears critical in many biological processes. Small vesicles released from cells have recently emerged as important mediators of inter-cellular communication. These vesicles, that have been termed "extracellular vesicles (EVs)", are inclusive of exosomes released from the endosomal cell-membrane compartment and of microvesicles released from the cell surface by plasma membrane budding. The EV content of proteins, lipids and nucleic acids varies with the cell of origin and, after incorporation into recipient cells, they may transfer information which may change the phenotype and function of recipient cells.

Several studies have addressed the role of EVs in physiological and pathological conditions based on their biological activity and molecular constituents. Moreover, since EVs retain the signature of the cell of origin and are present in all body fluids, their potential use as diagnostics in different pathological conditions has been suggested.

A fundamental issue remains how to isolate EVs from cultured cells to study their biological functions or from biological fluids for diagnostic purposes. Since foetal bovine serum frequently used for cell culture is enriched in EVs, the *in vitro* experiments require the use of EVs-depleted serum. The isolation of EVs from body fluids, on the other hand, has to face the complexity due to the concomitant presence of EVs of different cellular origin. Therefore, in order to identify a potential biomarker, it is critical to discriminate cellular origin on the basis of EV molecular expression or content, by proteomic or genomic analysis. After removal of cell debris by centrifugation, three main methods are conventionally used for isolation of EVs, namely differential ultracentrifugation in the absence or presence of sucrose gradient, size exclusion chromatography and immune affinity. All these methods have some advantages, which are mainly related to the possibility to discriminate between different EV populations, and concerns, which are related to the risk of damaging vesicles during purification with loss of biological activity, to the need of a sufficiently large sample and to the efficiency of isolation.

Moreover, polymeric precipitation of EVs has been suggested as an alternative method mainly focused on the evaluation of RNA and protein content. The polymeric precipitation methods are based on the formation of a mesh-like net, which embeds EVs with a size ranging from 60 to 180 nm. Such methods may be applied either to culture media or to body fluids. In particular, polymeric precipitation methods may have the advantage for detection of biomarkers in vesicles derived from small biological samples.

Currently, the "gold standard" methods of EV purification are the differential ultracentrifugation or the density gradient ultracentrifugation. These methods, however, are influenced by several parameters difficult to standardize such as viscosity of solutions, rotor type, centrifugal radius and g force. In addition, the integrity of EVs after prolonged high speed ultracentrifugation may be damaged. In fact, membrane debris were observed by electron microscopy and difficulty in recovering RNA and exosomal proteins has been reported.

Several other approaches to EV purification have been investigated. The size exclusion chromatography may have an advantage on ultracentrifugation in maintaining EVs integrity, since with this method EVs are not subjected to shear stress. Filtration with membranes with appropriate pores is also an alternative, but it does not guarantee removal of several small contaminants and does not avoid loss of EVs by binding to membranes. Immunoaffinity purification may isolate specific exosome subtypes maintaining integrity of their cargo.

A limitation of most of these techniques is the efficiency in the recovery of sufficient amounts of EVs starting from small biological samples.

In order to overcome the drawbacks of the prior art, in particular the complexity and expensiveness of the above-mentioned prior art methods, their low efficiency in EV recovery, the possible presence of contaminants in the isolated EVs and the risk of damaging EV membranes, the present invention provides a method of capturing extracellular vesicles (EVs) from a biological fluid sample as defined in appended claim 1. The use of a solid surface coated with a polycationic substance selected from protamine salts for detecting or quantifying the extracellular vesicles (EVs) in a biological fluid sample also falls within the scope of the present invention.

Further features and advantages of the invention are defined in the dependent claims.

The method of capturing extracellular vesicles (EVs) according to the present invention results from the finding that EVs display a negative charge, which allows them to interact with positive-charged molecules.

Solid surfaces coated with protamine salt are known in the prior art. For example, Elisa plates coated with protamine sulfate are commercially available (Cosmo Bio Co., Ltd., Japan) and are used for binding DNA for quantification. However, to the inventors' knowledge, their use for capturing and detecting EVs has never been disclosed in the prior art. The inventors observerd improved results of EV precipitiation with protamine hydrochloride over other polycationic salts. A solid surface preferably selected from the group consisting of a microtiter plate, a column, a magnetic bead and a non-magnetic bead coated with protamine hydrochloride is therefore also an object of the invention.

Protamine coated on a solid surface was found to bind EVs from biological fluid samples without the need of further reagents or processing steps. Based on this finding, the present inventors also developed the immunoassay method for detecting or quantifying extracellular vesicles (EVs) in a biological fluid sample as defined in independent claim 3 and the related kit as defined in appended claim 7.

The methods and kit of the present invention have the merit of simplicity and avoid the requirement of expensive equipment and may be used for an efficient detection and quantification of EVs from small biological samples.

The method of capturing extracellular vesicles (EVs) from a biological fluid sample according to the present invention comprises the step of contacting the biological fluid sample with a polycationic substance selected from protamine salts coated on a solid surface, whereby the extracellular vesicles (EVs) which are present in the biological fluid sample are bound on the coated solid surface.

A polycationic substance is a polycationic polymer which is a polymeric molecule having positive charges in multiple places.

Protamine salts are suitable for clinical applications. The most preferred form of protamine is protamine hydrochloride.

The solid surface for use in the method of the invention is preferably selected from the group consisting of a microtiter plate, a column, a membrane, fibrous web, a magnetic bead and a non-magnetic bead. A microtiter plate, such as a 96 wells ELISA plate, is most preferred, particularly within the context of diagnostic applications.

The inventors employed the method of the invention to capture EVs from both biological fluids and from cell culture conditioned media. Accordingly, the method of the invention can be used for capturing EVs from any biological fluid or cell culture conditioned medium, such as for example from blood, serum, plasma, saliva, urine, cerebrospinal fluid, milk or from a cell culture conditioned medium, preferably an adult stem cell culture conditioned medium, more preferably a mesenchymal stem cell culture conditioned medium or a liver pluripotent progenitor cell culture conditioned medium.

As mentioned above, the EVs-capturing method according to the invention has the advantage that EVs are bound on the solid surface quite easily, without the need of further reagents or processing steps.

Therefore, the capturing method according to the invention can be conveniently used in an immunoassay method for detecting or quantifying extracellular vesicles (EVs) in a biological fluid sample.

The immunoassay method of the invention comprises the steps of:
(i) contacting the biological fluid sample with a solid surface coated with a polycationic substance selected from protamine salts, whereby the extracellular vesicles (EVs) which are present in the biological fluid sample are bound on the coated solid surface;
(ii) removing the unbound extracellular vesicles (EVs);
(iii) adding an antibody capable of binding to the extracellular vesicles (EVs) bound on the solid surface coated with the polycationic substance, thereby forming an immunocomplex;
(iv) detecting or quantifying the immunocomplex.

The polycationic substance and the solid surface which are preferably used in the immunoassay method of the invention are as defined above with reference to the capturing method.

According to a preferred embodiment of the immunoassay method of the invention, the antibody capable of binding to the extracellular vesicles (EVs) bound on the solid surface coated with the polycationic substance selected from protamine salts is labelled with a detectable label. For example, the antibody is a biotin-conjugated primary antibody and the immunocomplex resulting from the binding of the primary antibody to the EVs captured on the solid surface is detected by means of a Streptavidin HRP-conjugate. However, the person skilled in the art is able to select and use other labelling and detection means which are known per se in the art.

The scope of the invention also comprises a kit suitable for carrying out the immunoassay method of the invention, the kit comprising (i) a solid surface coated with a polycationic substance, as described above; and (ii) an antibody capable of binding to extracellular vesicles (EVs) bound on the solid surface coated with the polycationic substance, as described above. The kit of the invention may also comprise suitable detection means for detecting the immunocomplex resulting from the binding of the primary antibody to the EVs captured on the solid surface.

In another aspect of the invention the polycationic substance, as described above, which is bound to the solid surface may serve as a ligand for adsorption of EV from biological liquids. In such cases the solid surface is preferably selected from the group of a column, a membrane, a fibrous web and adsorber beads. In such an application the polycationic substance bound to the solid surface is part of a filter or filter device.

A specific application of the method of the invention and of the filter and filter devices according to the invention relates to the removal of extracellular vesicles from patients' plasma or blood by adsorption.

The method of the invention for the adsorption of extracellular vesicles can be applied either ex vivo or in vivo, such as in a continuous extracorporeal circuit from blood or plasma in a way similar to common adsorption apheresis therapy. A specific application relates to the removal of extracellular vesicle associated with diseases mediated by extracellular vesicles, as described in WO2007/103572 or in EP2495025.

WO2007/103572 describes methods of removing micro-vesicular particles, including specifically tumour-associated exosomes, from the systemic circulation of a subject with the goal of reversing antigen-specific and antigen-nonspecific immune suppression. The removal of said micro-vesicular particles is carried out in an extracorporeal circulation device including a hollow fibre filter which allows passage of blood cells through the lumen of the hollow and allows the diffusion of the micro-vesicles to the exterior through the hollow fibre tubular membrane; in order to allow such a diffusion, the pores of the membrane of the hollow fibres are of a diameter sufficient to allow particles ranging from the size of 20 nm to 500 nm in diameter. In one embodiment, agents capable of binding the micro-vesicles, such as antibodies, proteins or aptamers are immobilised on the porous hollow fibre membrane or specifically on the porous exterior of the membrane.

EP2495025 discloses a filter and filter device for a biological fluid. The filter is made from one or more layers of a non-woven fibrous web which includes fibers having a surface of contact with the biological fluid which comprises polymeric materials which are conventionally used in filters for leucocyte or platelet depletion. The non-woven fibrous web has a CWST preferably in the range of from about 40 to about 80 dynlcm. The filters of EP2495025 are used e.g. for the removal of sepsis-associated microvesicles from patients' plasma or blood.

Within the present invention, diseases mediated by extracellular vesicles - comprise cancer, sepsis, SIRS (Systemic Inflammatory Response Syndrome), autoimmune diseases, inflammatory diseases, neurological and neurodegenerative diseases, Parkinson's disease, Alzheimer's disease. The paper Corrado C. et al., Int. J. Mol. SCi. 2013, 14, 5338-5366 provides an overview of Ev-mediated diseases.

Continuous extracorporeal circuit for adsorption apheresis therapy are well known in the art. In continuous extracorporeal circuit care need to be taken that the patients blood does not coagulate in the extracorporeal circuit. Heparin is used commonly as an anti-coagulant. Since polycationic substances, in particular protamine, are known to bind heparin heparin-independent anti-coagulation methods are preferred. Local anti-coagulation of the extracorporeal circuit by citrate and an calcium as known from WO 91/06326 is a preferred method.

The method of adsorption can also be applied as a step in purification and concentration wherein the step of adsorption is followed by the elution of the extracellular vesicles from the filter for further use.

The following example is provided by way of illustration only and is not intended to limit the scope of the invention as determined by the appended claims.

### EXAMPLE

### ELISA test on protamine-coated plates.

The inventors set up an innovative ELISA test to quantify EVs in biological fluids and cell supernatant based on protamine binding.

### Methods

Generation of standard curves. EVs obtained by ultracentrifugation (1000.000 g, 2 hours) were loaded on protamine coated plates at decreasing concentration (starting from 2000 x 108 EVs). Wells were filled with 50 µl of sample and left overnight at 37°C, to dry. The next day, an anti-CD133 or an anti-CD24 antibodies (both biotin-conjugated) was added at a dilution of 1:50, followed by gentle rocking for 1h at room temperature (RT); a Streptavidin HRP-conjugated was used 1:30000 and incubated for 1h at RT. The absorbance was read in a spectro-photometer using 450nm as the primary wave length and 655 nm as reference wavelength.

### Results

### 1. Calibration lines of pooled EVs

Two markers of urinary vesicles CD133 and CD24 were used to generate standard curves. The analysis of the absorbance value for the CD133 expression correlated with the EV concentration as measured by NTA, with a R² value very close to 1.

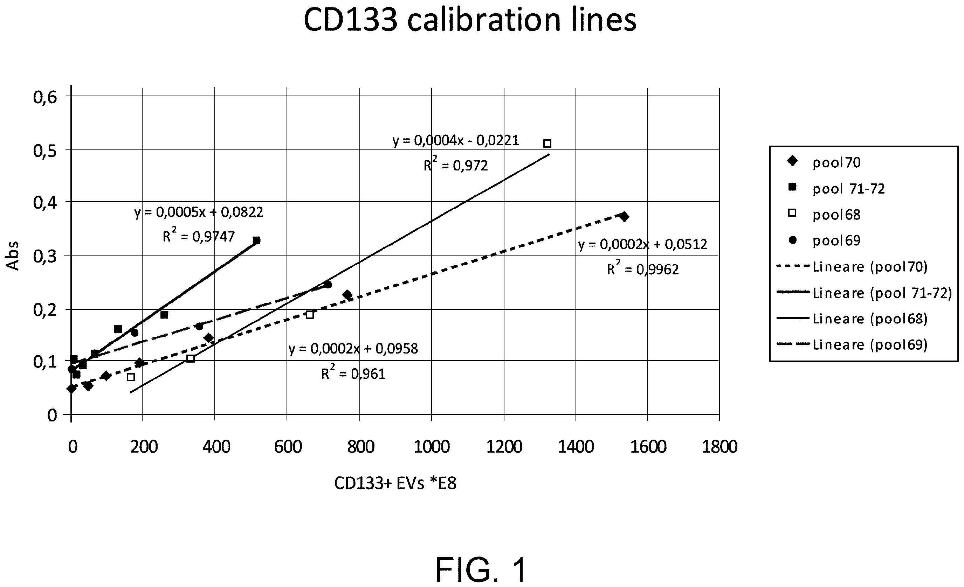

Figure 1 shows the CD133 calibration lines obtained with four different pools of EVs obtained by ultracentrifugation.

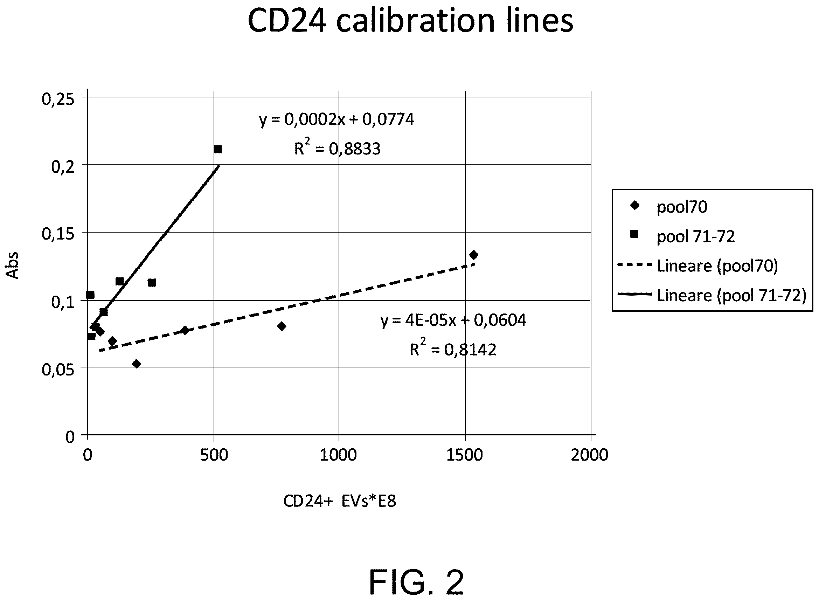

Similarly, the presence of CD24⁺ EVs correlated with the number of vesicles. Figure 2 shows the CD24 calibration lines obtained with two different pools of EVs obtained by ultracentrifugation.

### 2. Sample analysis

To evaluate the possibility to analyse the CD133 EVs in urine, a sample of undiluted total urine (50 µl) was tested in the ELISA assay (n=3). All samples gave a positive value in the range of the absorption line. We next compared the CD133⁺ EVs measured by cytofluorimetric analysis with the value measured with the ELISA assay. Table 1 shows the number of CD133 EVs as result of the ELISA test and the number of EVs in the same volume as calculated by ultracentrifugation, followed by NTA and cytofluorimetric analysis. Table 1. Number of CD133 EVs*10⁸ as result of the ELISA test (calculated with the absorbance on the calibration line obtained by NTA and cytofluorimetric analysis) and the number of EVs in the same volume as calculated by ultracentrifugation, followed by NTA and cytofluorimetric analysis.

| | ELISA | FACS + NTA |
|---|---|---|
| *1* | 395,25 | 1071,58 |
| *2* | 1025,75 | 1109,98 |
| *3* | 959 | 1103,11 |

Results show a good correlation.

## Claims

1. A method of capturing extracellular vesicles (EVs) from a biological fluid sample, the method comprising the step of contacting the biological fluid sample with a solid surface coated with a polycationic substance selected from protamine salts, whereby the extracellular vesicles (EVs) which are present in the biological fluid sample are bound on the coated solid surface.

2. The use of a solid surface coated with a polycationic substance selected from protamine salts, for detecting or quantifying the extracellular vesicles (EVs) in a biological fluid sample.

3. An immunoassay method of detecting or quantifying extracellular vesicles (EVs) in a biological fluid sample, comprising the steps of:
(i) contacting the biological fluid sample with a solid surface coated with a polycationic substance selected from protamine salts, whereby the extracellular vesicles (EVs) which are present in the biological fluid sample are bound on the coated solid surface;
(ii) removing the unbound extracellular vesicles (EVs);
(iii) adding an antibody capable of binding to the extracellular vesicles (EVs) bound on the solid surface coated with the polycationic substance, thereby forming an immunocomplex;
(iv) detecting or quantifying the immunocomplex.

4. The immunoassay method according to claim 3, wherein the antibody capable of binding to the extracellular vesicles (EVs) bound on the solid surface coated with the polycationic substance is labeled with a detectable label.

5. The immunoassay method according to claim 3 or 4, wherein the antibody capable of binding to the extracellular vesicles (EVs) bound on the solid surface coated with the polycationic substance is an anti-CD133 antibody or an anti-CD 24 antibody.

6. The immunoassay method according to any of claims 3 to 5, for the *in vitro* diagnosis of a disease.

7. A kit for detecting or quantifying extracellular vesicles (EVs) in a biological fluid sample, comprising:
(i) a solid surface coated with a polycationic substance selected from protamine salts; and
(ii) an antibody capable of binding to extracellular vesicles (EVs) bound on the solid surface coated with the polycationic substance.

8. The kit according to claim 7, wherein the antibody capable of binding to the extracellular vesicles (EVs) bound on the solid surface coated with the polycationic substance is labeled with a detectable label.

9. The kit according to claim 7 or 8, wherein the antibody capable of binding to the extracellular vesicles (EVs) bound on the solid surface coated with the polycationic substance is an anti-CD133 antibody or an anti-CD 24 antibody.

10. The kit according to any of claims 7 to 9, for use in the *in vitro* diagnosis of a disease.

11. The method of capturing extracellular vesicles (EVs) according to claim 1, or the use according to claim 2, or the immunoassay method according to any of claims 3 to 6, or the kit according to any of claims 7 to 10, wherein the salt is a hydrochloride.

12. The method of capturing extracellular vesicles (EVs) according to claim 1 or 11, or the use according to claim 2 or 11, or the immunoassay method according to any of claims 3 to 6 or 11, or the kit according to any of claims 7 to 11,wherein the solid surface is selected from the group consisting of a microtiter plate, a column, a membrane, fibrous web, a magnetic bead and a non-magnetic bead.

13. The method of capturing extracellular vesicles (EVs) according to claim 1 or 11 or 12, or the use according to claim 2 or 11 or 12, or the immunoassay method according to any of claims 3 to 6 or 11 or 12, or the kit according to any of claims 7 to 12,wherein biological fluid is selected from the group consisting of blood, serum, plasma, saliva, urine, cerebrospinal fluid, milk and the conditioned medium of a cell culture, wherein the cell culture is preferably an adult stem cell culture, more preferably a mesenchymal stem cell culture or a liver pluripotent progenitor cell culture.

## Patentansprüche

1. Verfahren zur Erfassung extrazelluläre Vesikel (EVs) aus einer biologischen Fluidprobe, wobei das Verfahren den Schritt des Kontaktierens der biologischen Fluidprobe mit einer festen Oberfläche umfasst, die mit einer polykationischen Substanz beschichtet ist, die aus Protaminsalzen ausgewählt ist, wodurch die extrazellulären Vesikel (EVs), die in der biologischen Fluidprobe vorhanden sind, an die beschichtete feste Oberfläche gebunden werden.

2. Verwendung einer festen Oberfläche, die mit einer polykationischen Substanz beschichtet ist, die aus Protaminsalzen ausgewählt ist, zum Detektieren oder Quantifizieren der extrazellulären Vesikel (EVs) in einer biologischen Fluidprobe.

3. Immunoassay-Verfahren zum Detektieren oder Quantifizieren extrazellulärer Vesikeln(EVs) in einer biologischen Fluidprobe, umfassend die Schritte:
(i) Kontaktieren der biologischen Fluidprobe mit einer festen Oberfläche, die mit einer polykationischen Substanz beschichtet ist, die aus Protaminsalzen ausgewählt ist, wodurch die extrazellulären Vesikel (EVs), die in der biologischen Fluidprobe vorhanden sind, an die beschichtete feste Oberfläche gebunden werden;
(ii) Entfernen der ungebundenen extrazellulären Vesikel (EVs);
(iii) Hinzufügen eines Antikörpers, der imstande ist, an die extrazellulären Vesikel (EVs) zu binden, die an die mit der polykationischen Substanz beschichtete feste Oberfläche gebunden sind, wodurch ein Immunkomplex gebildet wird;
(iv) Detektieren oder Quantifizieren des Immunkomplexes.

4. Immunoassay-Verfahren nach Anspruch 3, wobei der Antikörper, der imstande ist, an die extrazellulären Vesikel (EVs) zu binden, die an die mit der polykationischen Substanz beschichtete feste Oberfläche gebunden werden, mit einer detektierbaren Kennzeichnung gekennzeichnet ist.

5. Immunoassay-Verfahren nach Anspruch 3 oder 4, wobei der Antikörper, der an die extrazellulären Vesikel (EVs) binden kann, die an die mit der polykationischen Substanz beschichtete feste Oberfläche gebunden sind, ein Anti-CD 133-Antikörper oder ein Anti-CD24-Antikörper ist.

6. Immunoassay-Verfahren nach einem der Ansprüche 3 bis 5 für die In-vitro-Diagnose einer Krankheit.

7. Kit zum Detektieren oder Quantifizieren extrazellulärer Vesikel (EVs) in einer biologischen Fluidprobe, aufweisend:
(i) eine feste Oberfläche, die mit einer polykationischen Substanz beschichtet ist, die aus Protaminsalzen ausgewählt ist; und
(ii) einen Antikörper, der imstande ist, an extrazelluläre Vesikel (EVs) zu binden, die an die mit der polykationischen Substanz beschichtete feste Oberfläche gebunden sind.

8. Kit nach Anspruch 7, wobei der Antikörper, der imstande ist, an die extrazellulären Vesikel (EVs) zu binden, die an die mit der polykationischen Substanz beschichtete feste Oberfläche gebunden sind, mit einer detektierbaren Kennzeichnung gekennzeichnet ist.

9. Kit nach Anspruch 7 oder 8, wobei der Antikörper, der imstande ist, an die extrazellulären Vesikel (EVs) zu binden, die an die mit der polykationischen Substanz beschichtete feste Oberfläche gebunden sind, ein Anti-CD 133-Antikörper oder ein Anti-CD24-Antikörper ist.

10. Kit nach einem der Ansprüche 7 bis 9 zur Verwendung bei der In-vitro-Diagnose einer Krankheit.

11. Verfahren zur Erfassung extrazellulärer Vesikel (EVs) nach Anspruch 1 oder Verwendung nach Anspruch 2 oder Immunoassay-Verfahren nach einem der Ansprüche 3 bis 6 oder Kit nach einem der Ansprüche 7 bis 10, wobei das Salz ein Hydrochlorid ist.

12. Verfahren zur Erfassung extrazellulärer Vesikel (EVs) nach Anspruch 1 oder 11 oder Verwendung nach Anspruch 2 oder 11 oder Immunoassay-Verfahren nach einem der Ansprüche 3 bis 6 oder 11 oder Kit nach einem der Ansprüche 7 bis 11, wobei die feste Oberfläche ausgewählt ist aus der Gruppe bestehend aus einer Mikrotiterplatte, einer Säule, einer Membran, einem Faservlies, einem magnetischen Kügelchen und einem nichtmagnetischen Kügelchen.

13. Verfahren zur Erfassung extrazellulärer Vesikel (EVs) nach Anspruch 1 oder 11 oder 12 oder Verwendung nach Anspruch 2 oder 11 oder 12 oder Immunoassay-Verfahren nach einem der Ansprüche 3 bis 6 oder 11 oder 12 oder Kit nach einem der Ansprüche 7 bis 12, wobei biologisches Fluid ausgewählt ist aus der Gruppe bestehend aus Blut, Serum, Plasma, Speichel, Urin, Cerebrospinalflüssigkeit, Milch und dem konditionierten Medium einer Zellkultur, wobei die Zellenkultur vorzugsweise eine adulte Stammzellenkultur, ferner bevorzugt eine mesenchymale Stammzellenkultur oder eine pluripotente Leber-Progenitorzellenkultur ist.

## Revendications

1. Procédé permettant de capturer des vésicules extracellulaires (EV) depuis un échantillon de fluide biologique, le procédé comprenant l'étape de la mise en contact de l'échantillon de fluide biologique avec une surface solide revêtue d'une substance polycationique choisie parmi des sels de protamine, de telle manière que les vésicules extracellulaires (EV) qui sont présentes dans l'échantillon de fluide biologique soient liées sur la surface solide revêtue.

2. Utilisation d'une surface solide revêtue d'une substance polycationique choisie parmi des sels de protamine, permettant de détecter ou de quantifier les vésicules extracellulaires (EV) dans un échantillon de fluide biologique.

3. Procédé de dosage immunologique permettant de détecter ou de quantifier des vésicules extracellulaires (EV) dans un échantillon de fluide biologique, comprenant les étapes suivantes :
(i) la mise en contact de l'échantillon de fluide biologique avec une surface solide revêtue d'une substance polycationique choisie parmi des sels de protamine, de telle manière que les vésicules extracellulaires (EV) qui sont présentes dans l'échantillon de fluide biologique soient liées sur la surface solide revêtue ;
(ii) le retrait des vésicules extracellulaires (EV) non liées ;
(iii) l'ajout d'un anticorps capable de se lier aux vésicules extracellulaires (EV) liées sur la surface solide revêtue de la substance polycationique, en formant de ce fait un immunocomplexe ;
(iv) la détection ou la quantification de l'immunocomplexe.

4. Procédé de dosage immunologique selon la revendication 3, dans lequel l'anticorps capable de se lier aux vésicules extracellulaires (EV) liées sur la surface solide revêtue de la substance polycationique est marqué avec un marquage détectable.

5. Procédé de dosage immunologique selon la revendication 3 ou 4, dans lequel l'anticorps capable de se lier aux vésicules extracellulaires (EV) liées sur la surface solide revêtue de la substance polycationique est un anticorps anti-CD133 ou un anticorps anti-CD24.

6. Procédé de dosage immunologique selon l'une quelconque des revendications 3 à 5, destiné au diagnostic in vitro d'une maladie.

7. Kit permettant de détecter ou de quantifier des vésicules extracellulaires (EV) dans un échantillon de fluide biologique, comprenant :
(i) une surface solide revêtue d'une substance polycationique choisie parmi des sels de protamine ; et
(ii) un anticorps capable de se lier à des vésicules extracellulaires (EV) liées sur la surface solide revêtue de la substance polycationique.

8. Kit selon la revendication 7, dans lequel l'anticorps capable de se lier aux vésicules extracellulaires (EV) liées sur la surface solide revêtue de la substance polycationique est marqué avec un marquage détectable.

9. Kit selon la revendication 7 ou 8, dans lequel l'anticorps capable de se lier aux vésicules extracellulaires (EV) liées sur la surface solide revêtue de la substance polycationique est un anticorps anti-CD133 ou un anticorps anti-CD24.

10. Kit selon l'une quelconque des revendications 7 à 9, pour son utilisation dans le diagnostic *in vitro* d'une maladie.

11. Procédé permettant de capturer des vésicules extracellulaires (EV) selon la revendication 1, ou utilisation selon la revendication 2, ou procédé de dosage immunologique selon l'une quelconque des revendications 3 à 6, ou kit selon l'une quelconque des revendications 7 à 10, dans lequel le sel est un chlorhydrate.

12. Procédé permettant de capturer des vésicules extracellulaires (EV) selon la revendication 1 ou 11, ou utilisation selon la revendication 2 ou 11, ou procédé de dosage immunologique selon l'une quelconque des revendications 3 à 6 ou 11, ou kit selon l'une quelconque des revendications 7 à 11, dans lequel la surface solide est choisie dans le groupe se composant d'une plaque microtitre, une colonne, une membrane, une bande fibreuse, une bille magnétique et une bille non magnétique.

13. Procédé permettant de capturer des vésicules extracellulaires (EV) selon la revendication 1 ou 11 ou 12, ou utilisation selon la revendication 2 ou 11 ou 12, ou procédé de dosage immunologique selon l'une quelconque des revendications 3 à 6 ou 11 ou 12, ou kit selon l'une quelconque des revendications 7 à 12, dans lequel le fluide biologique est choisi dans le groupe se composant de sang, sérum, plasma, salive, urine, fluide cérébrospinal, lait et le milieu conditionné d'une culture de cellules, dans lequel la culture de cellules est de préférence une culture de cellules souches adultes, avec plus de préférence une culture de cellules souches mésenchymateuses ou une culture de cellules progénitrices pluripotentes du foie.
